# EUROPÄISCHE PATENTANMELDUNG

(11) **EP 0 768 078 A1**
(43) Veröffentlichungstag der Anmeldung: **16.04.1997**
(21) Anmeldenummer: 96115728.6
(22) Anmeldetag: 01.10.1996
(51) Int. Cl.: A61K 7/00, A61K 7/032, A61K 7/48

(54) **Verwendung von mit Metalloxiden dotierten Zinkoxiden für kosmetische Zwecke**

(30) Priorität: 09.10.1995 DE 19537480
(71) Anmelder: BASF Aktiengesellschaft, 67063 Ludwigshafen (DE)
(72) Erfinder: Pfrommer, Ellen, 67454 Hassloch (DE); Mronga, Norbert, Dr., 69221 Dossenheim (DE); Seeger, Oliver, Dr., 68199 Mannheim (DE)

(57) **Zusammenfassung**

Die Erfindung betrifft die Verwendung von mit Metalloxiden dotierten farbigen Zinkoxiden als Farbmittel bzw. UV-Absorber für kosmetische Zwecke.

## Beschreibung

Die vorliegende Erfindung betrifft die Verwendung von mit Metalloxiden dotierten farbigen Zinkoxiden für kosmetische Zwecke.

In der Kosmetik, speziell in der dekorativen Kosmetik, werden als Farbmittel häufig Pigmente eingesetzt. Pigmente zeichnen sich gegenüber Farbstoffen durch ein gutes Deckvermögen und gute Lichtstabilität aus. Für die Auswahl des Pigmentes sind neben den koloristischen Eigenschaften wie z.B. Farbton und Brillanz auch die toxikologische Bewertung von Bedeutung. Generell werden anorganische Pigmente dabei als toxikologisch unbedenklicher angesehen als organische Pigmente. Dies spiegelt sich auch in den entsprechenden gesetzlichen Bestimmungen in den USA wieder: die FDA hat anorganische Pigmente als "exempt from certification" zugelassen, organische Pigmente dagegen sind "subject to certification" und müssen daher chargenweise von der FDA geprüft und zugelassen werden. Außerdem sind in den USA für den Augenbereich viele anorganische Pigmente zugelassen, dagegen nur wenige organische Farbmittel.

Bezüglich der Koloristik werden jedoch häufig organische Pigmente bevorzugt, da sie eine höhere Brillanz aufweisen als die bekannten anorganischen Pigmente und damit außerdem ein größeres Spektrum an Farbtönen erreichbar ist. Bei den Gelb- bis Rottönen sind brillante Farbtöne bisher nur mit organischen Pigmenten erreichbar. Als anorganische Pigmente können Eisenoxide eingesetzt werden, diese sind jedoch deutlich stumpfer im Farbton.

Es bestand daher die Aufgabe, anorganische Pigmente bereitzustellen, die die vorteilhaften Eigenschaften von anorganischen Pigmenten (gute Echtheitseigenschaften, gute toxikologische Bewertung) mit hoher Farbbrillanz kombinieren.

Diese Aufgabe wurde gelöst durch die Verwendung von mit Metalloxiden dotierten farbigen Zinkoxiden für kosmetische Zwecke, wobei die dotierten farbigen Zinkoxide als Farbpigmente und/oder UV-Absorber wirken.

Solchermaßen dotierte Zinkoxide sind beispielsweise aus der US 2,028,980 oder der EP 482 444 bekannt.

In EP 482 444 wird ein besonders gut geeignetes Verfahren zur Herstellung dotierter Zinkoxide beschrieben, das zu besonders brillanten Farbpigmenten führt, und das dadurch gekennzeichnet ist, daß man die Oxide, Carbonate, Hydroxide und/oder Hydroxicarbonate des Zinks in Gegenwart von Wasser und von Oxiden, Carbonaten, Hydroxiden, Hydroxicarbonaten, Oxalaten oder Formiaten der Dotierungsmetalle mit Ameisensäure und/oder Oxalsäure zu den entsprechenden Formiaten und/oder Oxalaten umsetzt, zu einer Paste verarbeitet, die Paste trocknet, vermahlt und bei Temperaturen von 700 bis 1100°C in einer Inertgasatmosphäre calciniert.

Die nach den oben beschriebenen Verfahren hergestellten Zinkoxide sind besonders gut geeignet für die erfindungsgemäße Verwendung.

Als Dotierungsmetalle eignen sich besonders Ca, Fe, Mg und Mn in Form der zweiwertigen Metalloxide.

Ganz besonders ist Mn bevorzugt, das je nach Dotierungsmenge gelbe bis tiefrote Farbpigmente liefert.

Auch Mischungen von Dotierungsmetallen, z.B. Mn und Mg, sind gut geeignet für die Herstellung der dotierten Zinkoxide.

Für die Herstellung der mit Metallionen, insbesondere unter Verwendung von Metalloxiden dotierten farbigen Zinkoxide wird ausdrücklich auf die EP 482 444 und dort aufgeführten Literaturstellen verwiesen.

Die Herstellung der dotierten Zinkoxide wird bevorzugt so durchgeführt, daß man die Oxide, Carbonate, Hydroxide und/oder Hydroxicarbonate des Zinks in Gegenwart von Wasser und von Oxiden, Carbonaten, Hydroxiden, Hydroxicarbonaten, Oxalaten oder Formiaten der Dotierungsmetalle mit Ameisensäure und/oder Oxalsäure in einer mindestens für die Überführung der genannten Oxide, Carbonate, Hydroxide und/oder Hydroxicarbonate zu Formiaten oder Oxalaten ausreichenden Menge zu einer Paste verarbeitet, die Paste trocknet, vermahlt und bei Temperaturen von 700 bis 1100°C in einer Inertgasatmosphäre calciniert.

Als Metalloxide, mit denen das Zinkoxid dotiert werden kann, kommen insbesondere die zweiwertigen Oxide des Ca, Fe, Mn und Mg in Frage.

Das Herstellverfahren kann so durchgeführt werden, daß man das Oxid, Hydroxid, Carbonat und/oder Hydroxicarbonat des zweiwertigen Zinks sowie die entsprechenden Verbindungen der zweiwertigen Dotierungsmetalle in Gegenwart von Wasser mit Ameisensäure und/ oder Oxalsäure zu den entsprechenden Formiaten und/oder Oxalaten umsetzt. Die Wassermenge wird dabei so bemessen, daß die Mischung eine pastenförmige Konsistenz aufweist. Im allgemeinen ist dies bei Wassergehalten von 20 bis 45 Gew.-%, bezogen auf die übrigen Einsatzstoffe der Fall. Die Umsetzung kann bei Temperaturen von 10 bis 90°C durchgeführt werden. Die Säuren werden mindestens in solchen Mengen eingesetzt, die stöchiometrisch erforderlich sind, um die eingesetzten Metallverbindungen in die entsprechenden Oxalate bzw. Formiate zu überführen. Ein Überschuß an Säure über die stöchiometrisch erforderliche Menge schadet nicht, sollte aber zweckmäßig 10 % nicht übersteigen.

Gemäß einer weiteren Variante des Herstellverfahrens kann man auch schrittweise in der Weise vorgehen, daß man die Oxide, Carbonate, Hydroxide und/oder Hydroxicarbonate der zweiwertigen Dotierungsmetalle mit der Ameisensäure und/oder Oxalsäure in Gegenwart von Wasser umsetzt und dann die entstandene Lösung die Verbindungen des Zinks einträgt und umsetzt.

Auf eine Filtration oder auf das Waschen des in der ersten Stufe erhaltenen Vorproduktes kann verzichtet werden, weil die eingesetzten Wassermengen gering sind und bei der Umsetzung neben den Metallformiaten bzw. -oxalaten lediglich Verbindungen entstehen, die bei der nachfolgenden Trocknung oder Calcinierung ohnehin aus der Reaktionsmischung ausgetrieben werden.

Die Trocknung wird üblicherweise bei Temperaturen von 50 bis 100°C durchgeführt. Anschließend an die Trocknung wird das Produkt gemahlen und schließlich unter Inertgasatmosphäre, wie in einem Stickstoff- oder Kohlendioxidstrom bei Temperaturen von 700 bis 1100°C gebrannt. Ein Zusatz von Reduktionsmitteln erübrigt sich, da die Formiate bzw. Oxalate selbst reduzierend wirken und somit eine unerwünschte Oxidation der zweiwertigen Dotierungsmetalloxide unterbleibt.

Die so hergestellten Pigmente eignen sich für die Farbgebung von dekorativen Körperpflegemitteln, insbesondere von Augenpflegemitteln wie Lidschatten, Eyeliner, Wimperntusche, aber auch von Lippenstiften, Make-up und Nagellacken.

Neben dem Einsatz als Farbmittel wird Zinkoxid auch als UV-Absorber eingesetzt. Dabei wird bisher das herkömmliche, weiße Zinkoxid verwendet. Mit dem dotierten Zinkoxid besteht nun die Möglichkeit, ein anorganisches Pigment herzustellen, das zum einen als buntes Farbmittel und zum anderen als UV-Absorber eingesetzt werden kann.

Die vorliegende Erfindung betrifft auch kosmetische Mittel, enthaltend mit Metallionen dotierte farbige Zinkoxide.

Die folgenden Beispiele dienen der weiteren Veranschaulichung der Erfindung.

### Beispiel 1

Herstellung eines Lippenstiftes mit Mg und Mn dotierten Zinkoxid (Sicopal). Gemäß der untenstehenden Rezeptur wurde eine Lippenstiftgrundmasse hergestellt, in die die in der Tabelle angegebene Menge und Art des Farbpigments eingearbeitet wurde. Es wurden vier Lippenstifte (A - D) erhalten.

| | |
|---|---|
| 3,0 % | Permulgin 3430 |
| 4,0 % | Permulgin 3450 |
| 3,0 % | Permulgin 2550 |
| 3,5 % | Permulgin 3220 |
| 3,5 % | Permulgin 4200 |
| 1,5 % | Cutina CP |
| 6,0 % | Vaseline |
| 4,0 % | Lanfrax |
| 2,0 % | Lanolin |
| 11,0 % | Softisan 100 |
| 47,0 % | Rizinusöl |

Als Farbpigmente wurden folgende dotierten Zinkoxide eingesetzt
- P1:: Sicopal Gelb FK 4165 (mit 2,9 Gew.-% Mn und 3.2 Gew.-% Mg dotiertes ZnO)
- P2:: Sicopal Orange FK 4055 (mit 7,0 Gew.-% Mn und 1,6 Gew.-% Mg dotiertes ZnO)
- *):: Sicomet Weiß E 171

**Tabelle**

| | A | B | C | D |
|---|---|---|---|---|
| Lippenstiftmasse (Gew.-%) | 85,0 | 94,5 | 85,0 | 94,5 |
| Farbpigment (Gew.-%) | 15,0 P1 | 5,0 P1 | 15,0 P2 | 5,0 P1 |
| | | 0,5 ^{*)} | | 0,5 ^{*)} |

### Beispiel 2

### Herstellung eines Make-up

Gemäß der nachstehenden Rezeptur wurde ein Make-up hergestellt.

| | |
|---|---|
| 2,0 % | Glycerinmonostearat |
| 2,0 % | Cetylalkohol |
| 2,0 % | Cremophor A6 |
| 2,0 % | Cremophor A 25 |
| 5,5 % | Miglyol 812 |
| 5,0 % | Paraffinöl |
| 4,5 % | 1,2-Propylenglykol |
| qs | Konservierungsmittel |
| 61,0 % | Wasser dest |
| 0,5 % | Sicovil Schwarz 85 |
| 15,0 % | Sicopal Gelb FK 4165 (bzw. Sicopal Orange FK 4055) |

### Beispiel 3

### Herstellung eines Lidschattens

Gemäß der untenstehenden Rezeptur wurde ein Lidschatten hergestellt:

| | |
|---|---|
| 20,0 % | Talkum |
| 10,1 % | Kartoffelstärke |
| 5,2 % | Magnesiumstearat |
| 16,1 % | Binder* |
| 48,6 % | Sicopal Gelb FK 4165 (bwz. Sicopal Orange FK 4055) |

| | |
|---|---|
| * Binder: 2,0 % Glycerinmonostearat 2,0 % Cetylalkohol 2,0 % Cremophor A 6 2,0 % Miglyol 812 5,0 % 1,2-Propylenglykol qs Konservierungsmittel 75,0 % Wasser | |

## Patentansprüche

1. Verwendung von mit Metalloxiden dotierten farbigen Zinkoxiden für kosmetische Zwecke.

2. Verwendung nach Anspruch 1, dadurch gekennzeichnet, daß man als Dotierungmittel Oxide des zweiwertigen Eisens, Mangans, Magnesiums und Calciums verwendet.

3. Verwendung nach Anspruch 1 oder 2, dadurch gekennzeichnet, daß man die mit Metalloxiden dotierten farbigen Zinkoxide als Farbpigmente in kosmetischen Zubereitungen verwendet.

4. Verwendung nach Anspruch 1 oder 2, dadurch gekennzeichnet, daß man die mit Metalloxiden dotierten farbigen Zinkoxide als UV-Absorber in kosmetischen Zubereitungen verwendet.

5. Verwendung nach Anspruch 1 oder 2, dadurch gekennzeichnet, daß man die mit Metalloxiden dotieren farbigen Zinkoxide als Farbpigmente in Augenpflegemitteln verwendet.

6. Verwendung nach Anspruch 1 oder 2, dadurch gekennzeichnet, daß man die mit Metalloxiden dotierten farbigen Zinkoxide als Farbpigmente in Make-up verwendet.

7. Verwendung nach Anspruch 1 oder 2, dadurch gekennzeichnet, daß man die mit Metalloxiden dotierten farbigen Zinkoxide als Farbpigmente in Lippenstiften verwendet.

8. Verwendung nach Anspruch 1 oder 2, dadurch gekennzeichnet, daß man die mit Metalloxiden dotierten farbigen Zinkoxide als Farbpigmente in Nagellacken verwendet.

9. Kosmetische Mittel, enthaltend mit Metallionen dotierte farbige Zinkoxide.
